# EUROPÄISCHE PATENTANMELDUNG

(11) **EP 3 457 453 A1**
(43) Veröffentlichungstag der Anmeldung: **20.03.2019**
(21) Anmeldenummer: 17190870.0
(22) Anmeldetag: 13.09.2017
(51) Int. Cl.: H01M 2/02, A61N 1/375, H01M 2/26

(54) **AKKUMULATOR UND VERFAHREN ZUR HERSTELLUNG DESSELBEN**

(71) Anmelder: Wyon AG, 9050 Appenzell Steinegg (CH)
(72) Erfinder: Wyser, Paul J., 9050 Appenzell (CH); Sutter, Remo, 9062 Lustmühle (CH)
(74) Vertreter: Keller & Partner Patentanwälte AG

(57) **Zusammenfassung**

Ein Akkumulators, insbesondere ein Kleinakkumulator, umfasst mindestens zwei positive Elektroden mit jeweils einem Kontaktierungsanschluss und mindestens zwei negative Elektroden mit jeweils einem Kontaktierungsanschluss. Des Weiteren umfasst der Akkumulator mindestens eine Kontaktierungsvorrichtung mit mindestens einem Kontaktierungselement, das zwischen zwei benachbarten Kontaktierungsanschlüssen angeordnet ist und die zwei benachbarten Kontaktierungsanschlüsse elektrisch leitend miteinander verbindet. Ferner umfasst der Akkumulator ein Gehäuse, das die mindestens zwei positiven Elektroden, die mindestens zwei negativen Elektroden und die mindestens eine Kontaktierungsvorrichtung vollständig aufnimmt. Der Akkumulator weist mindestens eine Ummantelung auf, die die mindestens eine Kontaktierungsvorrichtung umgibt.

## Beschreibung

### Technisches Gebiet

Die Erfindung betrifft einen Akkumulator, insbesondere einen Kleinakkumulator, zum Aufnehmen und Abgeben von elektrischer Energie. Ferner betrifft die Erfindung die Verwendung eines erfindungsgemässen Akkumulators in einem medizintechnischen Gerät, insbesondere in einem in einen menschlichen Körper implantierbaren medizinischen Gerät. Zudem betrifft die Erfindung ein Verfahren zur Herstellung des erfindungsgemässen Akkumulators.

### Stand der Technik

Akkumulatoren werden als eine netzunabhängige elektrische Energieversorgung bei einer Vielzahl von tragbaren und auch stationären Geräten eingesetzt. Bei Geräten mit einem hohem Energiebedarf, wie beispielsweise Hörgeräten, Medikamentenspendern und/oder Diagnostik-Geräten, können auch Primärzellen eingesetzt werden, die jedoch den Nachteil haben, dass sie im Gegensatz zu Akkumulatoren nicht wieder aufladbar sind und alle fünf bis zehn Tage ersetzt werden müssen.

Aus der DE 10 2009 060 800 A1 (VARTA, 2011) sind Knopfzellen mit gewickelten Elektroden und Metallgehäuse in Becherform für Akkumulatoren und nicht wieder aufladbare Batterien bekannt. In der Praxis sind solche Knopfzellen für Bauhöhen über 5 mm im Einsatz. Für kleinere Bauhöhen ist der Wickelaufbau wegen des zu kleinen aktiven Volumens nachteilhaft. Ausserdem sind Knopfzellen mit Metallgehäuse in der Formgebung eingeschränkt. Dies wiederum verhindert die optimale Ausnutzung eines vorhandenen Volumens in einem mit einer Knopfzelle betriebenen Gerät und reduziert folglich die Kapazität der Knopfzelle.

Die US 20140072860 A1 (SWISSBAT, 2014) offenbart einen Akkumulator mit Kunststoffgehäuse und gestapeltem Elektrodenaufbau. Die Ableiterfahnen der Anode und Kathode werden jeweils auf einem Metallblock positioniert und verschweisst. Nachteilhaft daran ist der relativ grosse Platzbedarf des Ableiterblocks und der Ableiterfahnen. Deshalb ist dieser Aufbau für kleine oder kleinste Akkumulatoren, wie beispielsweise Knopfzellen, weniger geeignet. Zudem ist eine automatische Stapelung der Elektroden wegen der verschieden langen und verschieden gewinkelten Ableiterfahnen sehr schwierig und teuer.

Aus der EP 1 100 138 A (Wyon AG, 2001) ist ein Akkumulator mit raumsparender und kundenspezifischer Bauform, Wickelelektroden, integrierter Schutz- und Ladeschaltung sowie Kunststoffgehäuse bekannt, um bei Anwendungsgebieten mit hohem Energiebedarf, wie beispielsweise bei Hörimplantaten, die traditionellen Zink-Luft Primärzellen ersetzen zu können. Der Stromanschluss wird durch Röhrchen gebildet, deren Abstützung am Gehäuseboden zu einem Raumverlust führt. Ausserdem eignet sich die elektrische Kontaktierung mittels in das Gehäuse hineinragender Stifte nicht für Geräte, deren Batterien häufig gewechselt werden müssen.

Daher schlägt die US 7,294,430 B2 (Wyon AG, 2007) eine mechanisch spannbare Verbindung und einen Kontaktierungsanschluss an der Aussenseite des Gehäuses zur einfachen Kontaktierung durch ein Gerät vor. Allerdings ist diese Konstruktion für Akkumulatoren mit gewickelten Elektroden und einer Bauhöhe von weniger als ca. 6 mm auf Grund des relativ kleinen aktiven Volumens ungeeignet.

Um auch bei Knopfzellen eine platzsparende Anordnung der Elektroden bei gleichzeitig hoher Leistungsfähigkeit zu erzielen, schlägt die US 8,685,560 (Wyon, 2014) gefaltete Elektroden in einem Metallgehäuse vor.

### Darstellung der Erfindung

Aufgabe der Erfindung ist es, einen dem eingangs genannten technischen Gebiet zugehörende Akkumulator zu schaffen, welcher eine besonders hohe Kapazität und/oder Energiedichte hat. Insbesondere ist es eine Aufgabe der Erfindung, den für das aktive Material zur Verfügung stehenden Raum bei vorgegebenen Aussenabmessungen des Akkumulators zu maximieren. Eine weitere Aufgabe der Erfindung besteht im Schaffen eines effizienten und kostengünstigen Herstellungsverfahrens für einen erfindungsgemässen Akkumulator. Ausserdem soll eine besonders effektive Verwendung für einen erfindungsgemässen Akkumulator bereitgestellt werden.

Die Lösung der Aufgabe ist durch die Merkmale des Anspruchs 1 definiert. Gemäss der Erfindung umfasst ein Akkumulator, insbesondere ein Kleinakkumulator, zum Aufnehmen und Abgeben von elektrischer Energie, mindestens zwei positive Elektroden mit jeweils einem Kontaktierungsanschluss und mindestens zwei negative Elektroden mit jeweils einem Kontaktierungsanschluss auf. Zudem umfasst der Akkumulator mindestens eine Kontaktierungsvorrichtung mit mindestens einem Kontaktierungselement, das zwischen zwei benachbarten Kontaktierungsanschlüssen angeordnet ist und die zwei benachbarten Kontaktierungsanschlüsse elektrisch leitend miteinander verbindet. Des Weiteren umfasst der Akkumulator ein Gehäuse, das die mindestens zwei positiven Elektroden, die mindestens zwei negativen Elektroden und die mindestens eine Kontaktierungsvorrichtung vollständig aufnimmt. Der Akkumulator weist mindestens eine Ummantelung auf, die die mindestens eine Kontaktierungsvorrichtung umgibt.

Die Ummantelung der mindestens einen Kontaktierungsvorrichtung ermöglicht einen sehr platzsparenden Aufbau derselben, weil die übliche platzraubende Abwinkelung und direkte Verschweissung aller Kontaktierungsanschiüsse der Elektroden entfällt. Die Ummantelung dient als Führung und/oder Halterung für die Kontaktierungselemente, die daher sehr klein ausgeführt werden können und viel weniger Platz beanspruchen als die übliche Abwinkelung und direkte Verschweissung aller Kontaktierungsanschlüsse. Dadurch steht im Inneren des Gehäuses des Akkumulators mehr Platz für das aktive Elektrodenmaterial, auch aktives Material genannt, zur Verfügung. Der Akkumulator kann folglich eine höhere Kapazität und/oder Energiedichte haben als ein Akkumulator ohne eine Ummantelung der mindestens einen Kontaktierungsvorrichtung. Zudem kann die Ummantelung die mindestens eine Kontaktierungsvorrichtung vor unerwünschten Ablagerungen schützen. Die Ummantelung kann beispielsweise eine mit den negativen Elektroden verbundene Kontaktierungsvorrichtung vor Lithium Ablagerungen schützen. Eine Kontaktierungsvorrichtung mit Ummantelung ist bereits ab einer Bauhöhe des Akkumulators von 1 mm herstellbar. Versuche haben gezeigt, dass mit einem erfindungsgemässen Akkumulator der Energieinhalt gegenüber einem herkömmlichen Akkumulator um 10 % - 20 % gesteigert werden kann.

Unter einem Akkumulator ist ein wieder aufladbarer Speicher für elektrische Energie auf elektrochemischer Basis zu verstehen. Ein einzelnes wieder aufladbares Speicherelement wird Sekundärelement oder Sekundärzelle genannt. Im Gegensatz dazu ist eine Primärzelle nicht oder nur sehr begrenzt wieder aufladbar. Ein Akkumulator kann aus einer oder mehreren Sekundärzellen bestehen. Falls der Akkumulator aus mehreren Sekundärzellen besteht, lassen sich diese elektrisch zusammenschalten, und zwar entweder in Reihenschaltung zur Steigerung der nutzbaren elektrischen Spannung, oder in Parallelschaltung zur Steigerung der nutzbaren Kapazität.

Kleinakkumulatoren sind Akkumulatoren mit einem Volumen von weniger als 30 cm³, insbesondere mit einem Volumen kleiner als 10 cm³, und im ganz Besonderen mit einem Volumen von kleiner als 1 cm³. Kleinakkumulatoren können Knopfzellen sein. Knopfzellen können eine runde Form haben, wobei ihr Durchmesser grösser ist als ihre Höhe. Kleinakkumulatoren können aber auch quaderförmig sein oder eine beliebige, gerätespezifische Form haben, wie beispielsweise eine Hufeisenform oder eine Tropfenform. Unter einer gerätespezifischen Form ist eine Form zu verstehen, die dem für einen Akkumulator verfügbaren Raum in dem Gerät entspricht, in dem der Akkumulator eingesetzt werden soll. Kleinakkumulatoren können eine Bauhöhe vom 6 mm oder weniger haben, insbesondere von 5 mm oder weniger. Kleinakkumulatoren verfügen bevorzugt über eine einzige Sekundärzelle, die wiederum aus einer Vielzahl von parallelgeschalteten Elektroden bestehen kann. Kleinakkumulatoren können eine Nennspannung im Bereich von 1 V bis 5 V haben, insbesondere von 1.3 V bis 4 V, und im ganz Besonderen von 3 V bis 3.8 V.

Die Elektroden können schichtförmig ausgeführt sein. Jede Elektrode verfügt über aktives Material. An den negativen Elektroden ist Anodenmaterial angebracht und an den positiven Elektroden ist Kathodenmaterial angebracht. Dabei können die Elektroden einseitig oder beidseitig mit aktivem Material beschichtet sein. Die Elektroden können gestapelt angeordnet sein. In einem entsprechenden Stapel folgt auf eine negative Elektrode eine positive Elektrode. Dazwischen ist ein Separator angeordnet als Trennschicht. Ein Stapel von Elektroden kann eine Vielzahl von Elektroden aufweisen.

Der Kontaktierungsanschluss einer Elektrode kann ein leitendes Gitter, leitendes Trägerband und/oder eine leitende Folie, wie beispielsweise eine Fahne sein. Der Kontaktierungsanschluss ist mit einer positiven Elektrode oder mit einer negativen Elektrode elektrisch leitend verbunden und frei von aktivem Material. Der Kontaktierungsanschluss einer positiven oder einer negativen Elektrode kann gerade oder abgewinkelt sein. Ein abgewinkelter Kontaktierungsanschluss kann länger sein als ein gerader Kontaktierungsanschluss, hat aber ansonsten die gleiche Form.

Die Kontaktierungsvorrichtung stellt eine elektrisch leitende Verbindung zwischen den Kontaktierungsanschlüssen der Elektroden her. Vorzugsweise stellt die Kontaktierungsvorrichtung eine leitende Verbindung zwischen Elektroden her, die über das gleiche aktive Material verfügen. Eine Kontaktierungsvorrichtung kann also insbesondere die Kontaktierungsanschlüsse von positiven Elektroden miteinander verbinden, so dass die positiven Elektroden elektrisch parallel geschaltet sind, oder sie kann insbesondere die Kontaktierungsanschlüsse von negativen Elektroden elektrisch miteinander verbinden, so dass diese elektrisch parallel geschaltet sind. Die Kontaktierungsvorrichtung kann säulenförmig sein und/oder einen kaskadenförmigen Aufbau haben. Bevorzugt hat die Kontaktierungsvorrichtung einen runden, ovalen oder eckigen Querschnitt, beispielsweise einen viereckigen Querschnitt.

Das mindestens eine Kontaktierungselement kann ganz oder teilweise aus einem elektrisch leitenden Material sein. Bevorzugt umfasst das mindestens eine Kontaktierungselement bei Verwendung zusammen mit positiven Elektroden ein Metall, wie beispielsweise Aluminium, rostfreier vernickelter Stahl oder Titan. Bei Verwendung des mindestens einen Kontaktierungselementes zusammen mit negativen Elektroden umfasst das Kontaktierungselement bevorzugt ein Metall wie beispielsweise Kupfer, rostfreier vernickelter Stahl oder Titan. Alternativ und/oder ergänzend dazu kann das mindestens eine Kontaktierungselement auch ganz oder teilweise mit einem leitenden Material beschichtet sein. Das mindestens eine Kontaktierungselement kann elektrolytbeständig und/oder korrosionsbeständig sein. Das Kontaktierungselement kann einen runden, ovalen oder eckigen Querschnitt haben, beispielsweise kann es einen viereckigen Querschnitt haben.

Die Höhe eines Kontaktierungselementes entspricht vorzugsweise dem Abstand von zwei direkt benachbarten Kontaktierungsanschlüssen oder einem ganzzahligen Vielfachen davon. Beispielsweise können jeweils zwei direkt benachbarte Kontaktierungsanschlüsse zueinander abgewinkelt werden und abstandsfrei miteinander verbunden werden, also ohne dass ein Kontaktierungselement dazwischen ist. Die somit neu entstandenen benachbarten Kontaktierungsanschiüsse haben dann den doppelten Abstand wie direkt benachbarte, aber nicht abgewinkelte Kontaktierungsanschlüsse. Folglich hat das mindestens eine Kontaktierungselement dann auch die doppelte Höhe. Entsprechend können auch drei oder mehr direkt benachbarte Kontaktierungsanschiüsse zusammengefasst werden, so dass die Höhe des Kontaktierungselementes dann dem Dreifachen oder mehrfachen des Abstandes zweier direkt benachbarter Kontaktierungsanschiüsse entspricht.

Das Gehäuse weist vorzugsweise eine Gehäusedurchführung auf, um eine elektrisch leitende Verbindung zwischen der mindestens einen Kontaktierungsvorrichtung und einem äusseren Kontakt des Akkumulators zu ermöglichen. Dabei kann sich die mindestens eine Kontaktierungsvorrichtung in die Gehäusedurchführung erstrecken. Ein Gehäuse, das die mindestens eine Kontaktierungseirichtung vollständig aufnimmt, hat den Vorteil, dass bis auf die vorgenannte Gehäusedurchführung keine weitere Gehäusedurchführung erforderlich ist zur Aufnahme der mindestens einen Kontaktierungsvorrichtung, und daher potentielle Undichtigkeiten des Gehäuses minimiert werden können.

Das Gehäuse kann gasdicht, elektrolytbeständig, korrosionsbeständig und/oder elektrisch isolierend sein. Gasdichte Gehäuse ermöglichen eine lange Lebensdauer des Akkumulators, weil ein Ausgasen des Akkumulators nicht möglich ist und auch keine unerwünschten Stoffe in das Innere des Gehäuses hineindiffundieren können. Vorteilhafterweise kann das Gehäuse aus zwei Teilen bestehen, die bei der Fertigung des erfindungsgemässen Akkumulators gasdicht miteinander verbunden werden. Beispielsweise kann das Gehäuse aus einem Gehäusebecher und einem Gehäusedeckel bestehen.

Unter gasdicht ist erfindungsgemäss zu verstehen, dass Gasdiffusion, insbesondere Wasserdampfdiffusion, nicht stattfindet oder vernachlässigbar ist. Unter isolierend ist zu verstehen, das eine Leitfähigkeit von weniger als 10⁻⁶ S/m vorliegt. Insbesondere ist unter isolierend die Leitfähigkeit eines als Nichtleiter, oder eines als Isolator bekannten Stoffes oder Stoffgemisches zu verstehen.

Ein äusserer Kontakt des Akkumulators kann der Minuspol oder der Pluspol des Akkumulators sein. Der Minuspol und der Pluspol des Akkumulators können sich an einer Aussenseite des Gehäuses befinden und gut zugänglich sein. Die äusseren Kontakte können möglichst klein und flach ausgeführt werden und sich bevorzugt an einem Rande des Gehäuses befinden. Ein äusserer Kontakt kann aus rostfreiem vergoldetem Stahl, vergoldetem Messing, Kupfer, Titan oder Gold sein. An die äusseren Kontakte können Drähte angelötet oder angeschweisst werden, oder die äusseren Kontakte können direkt auf einer Leiterplatte montiert werden.

Die Ummantelung kann die Hälfte oder mehr als die Hälfte eines Umfangs der mindestens einen Kontaktierungsvorrichtung umgeben, insbesondere kann sie zwei Drittel oder mehr des Umfangs der mindestens einen Kontaktierungsvorrichtung umgeben. In jedem Fall lässt die Ummantelung einen Teil des Umfangs der Kontaktierungsvorrichtung frei, beispielsweise kann die Ummantelung ein Fünftel oder mehr als ein Fünftel des Umfangs der Kontaktierungsvorrichtung frei lassen. Dabei kann der Umfang der Kontaktierungsvorrichtung entlang der äusseren Umrandung des Querschnitts, wie zuvor erwähnt, ermittelt werden.

Die Ummantelung kann die Kontaktierungsvorrichtung so eng umschliessen, dass sich der Fliessweg für Ionen durch den Elektrolyten zur Kontaktierungsvorrichtung derart verlängert, dass praktisch keine Ionen durch den Elektrolyten zur Kontaktierungsvorrichtung gelangen und sich an der Kontaktierungsvorrichtung auch keine entsprechenden Materialablagerungen, wie beispielsweise Lithiumablagerungen, bilden können.

Die Wandstärke der Ummantelung kann in einem Bereich von 0.005 mm bis 1 mm liegen, im Besonderen in einem Bereich von 0.01 mm bis 0.6 mm, und im ganz besonderen in einem Bereich von 0.15 mm bis 0.4 mm.

In einer bevorzugten Ausführungsform weist die Kontaktierungsvorrichtung eine Einspannvorrichtung auf, die das mindestens eine Kontaktierungselement und die mindestens zwei benachbarten Kontaktierungsanschlüsse einspannt.

Dadurch wird erreicht, dass der elektrische Übergangswiderstand und/oder der elektrische Kontaktwiderstand zwischen dem mindestens einen Kontaktierungselement und den mindestens zwei benachbarten Kontaktierungsanschlüsse möglichst klein werden. Dies vermindert den Innenwiderstand des Akkumulators und erhöht dessen Wirkungsgrad.

Alternativ und/oder zusätzlich zur Einspannvorrichtung kann auch ein leitender Stab oder Draht an das mindestens eine Kontaktierungselement und die zwei benachbarten Kontaktierungsanschlüsse angeschweisst oder angelötet werden. Möglich sind auch Bondverbindungen oder Klebeverbindungen mit einem leitfähigen Kleber.

In einer weiteren bevorzugten Ausführungsform weisen die mindestens zwei benachbarten Kontaktierungsanschlüsse jeweils ein Loch auf. Das mindestens eine Kontaktierungselement ist eine Hülse. Die Einspannvorrichtung weist einen Stift und ein Gegenlager auf. Der Stift hat im Bereich eines ersten Endes einen Flansch und im Bereich eines zweiten Endes eine Verbindung mit dem Gegenlager. Der Stift führt durch die Löcher der mindestens zwei benachbarten Kontaktierungsanschlüsse und durch das mindestens eine Kontaktierungselement. Die mindestens zwei benachbarten Kontaktierungsanschlüsse und das mindestens eine Kontaktierungselement befinden sich zwischen dem Flansch und dem Gegenlager.

Dadurch lassen sich die Einspannvorrichtung und die Kontaktierungsvorrichtung besonders einfach fertigen. Der Stift kann zudem dazu dienen, eine einfache und zuverlässige elektrische Verbindung zu einem äusseren Kontakt des Akkumulators herzustellen.

Das mindestens eine Kontaktierungselement, insbesondere die mindestens eine Hülse, kann beispielsweise aus einem Metall wie Kupfer, Aluminium, Gold, Silber oder Titan sein. Diese Metalle haben eine gute Leitfähigkeit und sind korrosionsbeständig. Das mindestens eine Kontaktierungselement, insbesondere die mindestens eine Hülse, kann auch aus Metallverbindungen bestehen. Die mindestens eine Hülse kann einen Innendurchmesser zwischen 0.1 mm und 2 mm, im Besonderen zwischen 0.2 mm und 1 mm, im ganz Besonderen zwischen 0.4 mm und 0.8 mm, und im Speziellen zwischen 0.3 mm und 0.6 mm haben. Der Aussendurchmesser der mindestens einen Hülse beträgt dann zwischen 0.2 mm und 4 mm, beziehungsweise im Besonderen zwischen 0.4 mm und 2 mm, beziehungsweise im ganz Besonderen zwischen 0.6 mm und 1.5 mm.

Alternativ kann das mindestens eine Kontaktierungselement und/oder die mindestens eine Hülse auch aus einem oder mehreren nicht-metallischen Werkstoffen bestehen, wie beispielsweise leitendem Kunststoff. Das mindestens eine Kontaktierungselement kann beispielsweise auch ein oder mehrere organische Stoffe beinhalten, wie beispielsweise Kohlenstoff, insbesondere strukturellen Kohlenstoff.

Vorteilhafterweise besteht der Stift ganz oder teilweise aus einem leitfähigen Material. Bevorzugt besteht der Stift bei Verwendung zusammen mit positiven Elektroden aus einem Metall wie beispielsweise Aluminium, rostfreiem vernickelten Stahl oder Titan. Bei Verwendung des Stiftes zusammen mit negativen Elektroden besteht er bevorzugt aus einem Metall wie beispielsweise Kupfer, rostfreiem vernickelten Stahl oder Titan. Alternativ und/oder zusätzlich kann der Stift auch mit einem leitfähigen Material ganz oder teilweise beschichtet sein. Alternativ kann der Stift auch aus einem oder mehreren nicht-metallischen Werkstoffen bestehen, wie beispielsweise leitendem Kunststoff. Der Stift kann beispielsweise auch ein oder mehrere organische Stoffe beinhalten, wie beispielsweise Kohlenstoff, insbesondere strukturellen Kohlenstoff.

Bevorzugt hat der Stift einen runden, ovalen oder eckigen Querschnitt, beispielsweise kann er einen viereckigen Querschnitt haben. Falls der Stift einen runden Querschnitt hat, kann er einen Aussendurchmesser zwischen 0.1 mm und 2 mm, im Besonderen zwischen 0.2 mm und 1 mm, und im ganz Besonderen zwischen 0.4 mm und 0.8 mm haben. Bei anderen Querschnittsformen kann der Stift Querschnittsflächen haben zwischen 0.01 mm² und 3 mm², im Besonderen zwischen 0.03 mm² und 1 mm², und im ganz Besonderen zwischen 0.1 mm² und 0.3 mm². Vorteilhafterweise hat der Stift Aussenabmessungen, die im Wesentlichen den Innenabmessungen der mindestens einen Hülse entsprechen oder geringfügig kleiner sind als die Innenabmessungen der mindestens einen Hülse, damit sich die mindestens eine Hülse leicht auf den Stift aufschieben lässt. Bevorzugt ist der Aussendurchmesser oder die Aussenabmessungen des Stiftes entlang seiner Länge - mit Ausnahme des Flansches - konstant. Alternativ kann sich der Aussendurchmesser des Stiftes entlang seiner Länge auch ändern.

Alternativ können die Aussenabmessungen des Stiftes auch geringfügig grösser sein als die Innenabmessungen der mindestens einen Hülse. Die mindestens eine Hülse kann dann auf den Stift aufgepresst werden.

Das Gegenlager kann ein Kontaktierungselement und insbesondere eine Hülse sein. Beispielsweise kann eine aufgepresste Hülse ein Gegenlager bilden.

Alternativ zum Stift kann auch eine Röhre mit einem Längsschlitz eingesetzt werden. Statt der Hülsen können dann Abstandshalter ohne Durchgangsloch Verwendung finden. Durch den Längsschlitz in der Röhre können die Kontaktierungsanschlüsse der Elektroden in die Röhre eingebracht werden. Zwischen zwei benachbarten Kontaktierungsanschlüssen kann ein Abstandshalter in der Röhre angeordnet sein. Der Innendurchmesser der Röhre hat an einem Ende der Röhre eine Verjüngung, so dass ein Auflager für die Kontaktierungsanschlüsse und die Abstandshalter entsteht, analog zum Flansch in der bevorzugten Ausführungsform der Einspannvorrichtung mit Stift.

Alternativ und/oder zusätzlich zu der mindestens einen Hülse kann auch ein Kamm eingesetzt werden. Zwischen den Zacken des Kamms können die Kontaktierungsanschlüsse der Elektroden aufgenommen werden, so dass der Kamm und die Kontaktierungsanschlüsse elektrisch leitend miteinander verbunden sind, beispielsweise in Form einer Klemm- oder Quetschverbindung. Der Kamm hat den Vorteil, dass weder ein Stift noch eine Röhre benötigt wird. Fertigungstechnisch hat jedoch die bevorzugte Ausführungsform mit mindestens einer Hülse Vorteile.

In einer weiteren bevorzugten Ausführungsform ist die Verbindung des Stiftes mit dem Gegenlager ein Gewinde, eine Nietverbindung und/oder eine Schweissverbindung.

Solche Verbindungen sind sehr einfach herzustellen und sehr zuverlässig. Beispielsweise kann ein Kontaktierungselement, das sich an dem Ende des Stiftes befindet, das dem flanschseitigen Ende abgewandt ist, auf den Stift aufgeschraubt sein. Ein solches Kontaktierungselement kann alternativ auch mit dem Stift vernietet oder verschweisst sein.

Alternativ und/oder zusätzlich dazu kann die Verbindung des Stiftes mit dem Gegenlager auch durch Löten, Kleben und/oder eine kraftschlüssige Verbindung, wie beispielsweise eine Pressverbindung, erfolgen. In letzterem Fall wird das Gegenlager auf den Stift aufgepresst.

In einer weiteren bevorzugten Ausführungsform sind die Kontaktierungsanschlüsse der mindestens zwei positiven Elektroden benachbart, und/oder die Kontaktierungsanschlüsse der mindestens zwei negativen Elektroden sind benachbart.

Dadurch kann eine elektrische Parallelschaltung der mindestens zwei positiven Elektroden und/oder eine Parallelschaltung der mindestens zwei negativen Elektroden realisiert werden. Mit einer Parallelschaltung von Elektroden lässt sich eine Erhöhung der Kapazität und/oder der Energiedichte des Akkumulators erreichen.

Alternativ zu einer Parallelschaltung von Elektroden können die Kontaktierungsanschlüsse so angeordnet sein, dass ein Kontaktierungsanschluss einer positiven Elektrode der mindestens zwei positiven Elektroden und ein Kontaktierungsanschluss einer negativen Elektrode der mindestens zwei negativen Elektroden benachbart sind. In diesem Fall ist eine Serienschaltung von Elektroden realisierbar, wenn diese zu unterschiedlichen Sekundärzellen gehören. Dabei können auch Isolatoren dort zum Einsatz kommen, wo keine leitende Verbindung zwischen zwei benachbarten Kontaktierungsanschlüssen für eine Serienschaltung nötig ist, sondern zu einem Kurzschluss führen würde.

In einer weiteren bevorzugten Ausführungsform weist der Akkumulator zwei Kontaktierungsvorrichtungen auf, die beide vollständig im Gehäuse angeordnet sind.

Damit lässt sich gegenüber den Ausführungsformen mit nur einer Kontaktierungsvorrichtung ein noch platzsparender Aufbau erzielen. Dadurch, dass beide Kontaktierungsvorrichtungen vollständig im Gehäuse angeordnet sind, kann die Anzahl der Gehäusedurchführungen minimiert werden, und damit können auch potentielle Undichtigkeiten des Gehäuses minimiert werden.

Jede der zwei Kontaktierungsvorrichtungen kann eine Ummantelung aufweisen.

In einer weiteren bevorzugten Ausführungsform besteht die Ummantelung aus Kunststoff.

Eine Ummantelung aus Kunststoff ist einfach herstellbar, beispielsweise per Spritzguss. Eine Ummantelung aus Kunststoff kann elektrolytbeständig, korrosionsbeständig und/oder elektrisch isolierend sein. Falls der Akkumulator zwei Kontaktierungsvorrichtungen aufweist, können beide Ummantelungen aus Kunststoff bestehen und einteilig gefertigt werden, beispielsweise als ein brillenförmiges Kunststoffteil. Eine Ummantelung aus Kunststoff, oder zwei Ummantelungen aus Kunststoff können beispielsweise in ein Gehäuse aus Metall einsetzbar sein.

Vorteilhafterweise werden für die Ummantelung Kunststoffe wie flüssigkristaline Elastomere (LCP) oder Polyethylen (PE) eingesetzt. Die Ummantelung kann auch verstärkten LCP enthalten.

Bei Verwendung eines Gehäuses aus Metall kann zumindest eine Kontaktierungsvorrichtung, insbesondere wenn zwei Kontaktierungsvorrichtungen im Akkumulator Verwendung finden, elektrisch vom Gehäuse isoliert angeordnet sein, was durch mindestens eine Ummantelung aus Kunststoff erreicht werden kann.

Alternativ kann die Ummantelung auch aus Metall sein. Falls das Gehäuse aus zwei Teilen besteht, die jeweils auch aus Metall sind, kann die Ummantelung in einen Teil der zwei Teile des Gehäuses integriert werden. Bei zwei Ummantelungen kann in jeden Teil der zwei Teile des Gehäuses eine Ummantelung integriert werden.

In einer weiteren bevorzugten Ausführungsform besteht das Gehäuse aus Kunststoff.

Solche Gehäuse können einfach, kostengünstig und mit kundenspezifischen Abmessungen beispielsweise mittels Spritzguss hergestellt werden. Ein Gehäuse aus Kunststoff kann elektrolytbeständig, korrosionsbeständig und/oder elektrisch isolierend sein. Bei einem elektrisch isolierenden Gehäuse braucht kein Sicherheitsabstand zwischen der Gehäuseinnenwand und dem aktiven Material eingehalten werden, sondern das aktive Material kann das Gehäuse berühren, so dass mehr aktives Material in den Akkumulator eingebracht werden kann als bei einem Akkumulator mit elektrisch leitendem Gehäuse. Ebenso kann mit einer genauen Einpassung des Akkumulators in ein Gerät, was mit einem Gehäuse aus Kunststoff besonders einfach ist, nochmals zusätzlicher Energieinhalt gewonnen werden. Bei einem Gehäuse aus Kunststoff ist die Formgebung kaum eingeschränkt.

Vorteilhafterweise wird für das Gehäuse der Kunststoff LCP (flüssigkristaline Elastomere) eingesetzt. Das Gehäuse kann auch verstärkten LCP enthalten.

Das Gehäuse aus Kunststoff kann eine Wandstärke von weniger als 0.5 mm, im Besonderen von weniger als 0.3 mm, und im ganz Besonderen von 0.2 mm oder weniger aufweisen. Die Wandstärke spielt bei Kleinakkumulatoren eine wesentliche Rolle, da bei vorgegebenen Aussenabmessungen des Akkumulators eine Verringerung der Wandstärke dazu genutzt werden kann, mehr aktives Material in das Gehäuse einzubringen und damit die Kapazität des Akkumulators zu erhöhen.

Wenn nicht nur das Gehäuse, sondern auch die Ummantelung oder die zwei Ummantelungen aus Kunststoff bestehen, können die Ummantelung oder die zwei Ummantelungen in einen Teil des Gehäuses integriert werden, beispielsweise in einen Gehäusebecher. In diesem Fall können der Gehäusebecher und die eine Ummantelung einteilig fertigbar sein, und/oder der Gehäusebecher und die zwei Ummantelungen können einteilig fertigbar sein, was die Fertigung und/oder Montage des Akkumulators vereinfacht. Ausserdem stellt die Integration der Ummantelung oder der Ummantelungen in das Gehäuse eine weitere Platzersparnis dar, was zu einer Erhöhung des aktiven Materials und damit der Kapazität des Akkumulators genutzt werden kann.

Alternativ kann das Gehäuse auch aus Metall sein. Dadurch wird der Akkumulator sehr robust gegenüber mechanischen Einflüssen von aussen.

In einer weiteren bevorzugten Ausführungsform weist das Gehäuse eine an einer Aussenseite des Gehäuses angeordnete Öse auf.

Die Öse kann der Befestigung des Akkumulators in einem Gerät dienen, oder die Öse kann dazu genutzt werden, um etwas am Akkumulator zu befestigen.

Die Öse kann an einer Aussenseite eines Gehäusebodens, einer Aussenseite eines Gehäusedeckels oder einer Aussenseite einer Gehäuseseitenwand angeordnet sein. Es können auch mehrere Ösen an einer oder mehreren Aussenseiten des Gehäuses angebracht sein, beispielsweise an einer Aussenseite eines Gehäusebodens, einer Aussenseite eines Gehäusedeckels und/oder einer Aussenseite einer Gehäuseseitenwand.

Alternativ kann der Akkumulator auch ohne Öse oder mit einem anderen Befestigungsmittel ausgeführt werden.

In einer weiteren bevorzugten Ausführungsform weist mindestens eine der negativen Elektroden einen Stromableiter aus einem elektrisch leitenden nicht-metallischen strukturellen Material auf.

Ein Stromableiter aus einem elektrisch leitenden nicht-metallischen strukturellen Material hat den Vorteil, dass er korrosionsbeständig ist und zudem günstiger sein kann als beispielsweise ein korrosionsbeständiges Metall. Korrosionsbeständige Stromableiter führen zu einer verbesserten Lebensdauer des Akkumulators. Ausserdem ist es bei der Verwendung eines Stromableiters aus einem elektrisch leitenden nicht-metallischen strukturellen Material nicht erforderlich, metallische Kontaktierungselemente, wie beispielsweise metallische Anschlussfahnen, an den Elektroden anzubringen. Der Stromableiter aus einem elektrisch leitenden nicht-metallischen strukturellen Material kann an einem Bereich, wo beispielsweise kein aktives Material angebracht ist, als Kontaktierungsanschluss dienen. Dieser nicht-metallische Kontaktierungsanschluss und/oder mehrere nicht-metallische Kontaktierungsanschlüsse können direkt mit einem oder mehreren Kontaktierungselementen in Kontakt gebracht werden, um so eine Kontaktierungsvorrichtung zu bilden.

Das elektrisch leitende nicht-metallische strukturelle Material kann aus einem elektrisch leitenden Kunststoff und/oder einem elektrisch leitenden organischen Stoff oder Stoffgemisch bestehen, wie beispielsweise strukturellem Kohlenstoff. Struktureller Kohlenstoff unterscheidet sich von dem Kohlenstoff, der als aktives Material genutzt wird.

Bevorzugt kann der Stromableiter aus strukturellem Kohlenstoff als Folie oder Blatt ausgebildet sein.

Eine Folie und/oder ein Blatt aus strukturellem Kohlenstoff kann ein Trägerband bilden, das der Aufnahme des aktiven Materials dient.

Bevorzugt kann auch mindestens eine positive Elektrode einen Stromableiter aus strukturellem Kohlenstoff aufweisen.

Für weiterführende Einzelheiten betreffend Stromableiter aus einem elektrisch leitenden nicht-metallischen strukturellen Material wird auf die europäische Patentanmeldung mit dem Titel "Rechargeable Battery" des gleichen Anmelders mit dem gleichen Anmeldtag wie die vorliegende Patentanmeldung verwiesen.

Bevorzugt kann der Akkumulator eine wieder aufladbare Lithium oder Lithium Ion Batterie sein.

Ein weiterer Aspekt der Erfindung betrifft die Verwendung eines erfindungsgemässen Akkumulators. Der erfindungsgemässe Akkumulator wird in einem medizintechnischen Gerät, insbesondere in einem Hörgerät, und im ganz Besonderen in einem in einen menschlichen Körper implantierbaren medizinischen Gerät, verwendet.

Auf Grund der gesteigerten Kapazität des erfindungsgemässen Akkumulators kann die Zeitdauer zwischen Aufladungen verlängert werden, was den Komfort des Nutzers des Gerätes erhöht.

Medizintechnische Geräte können beispielsweise Diagnosegeräte, Hörgeräte, und/oder implantierbare Geräte wie beispielsweise implantierbare Pumpen oder Hörimplantate sein.

Ein weiterer Aspekt der Erfindung betrifft ein Verfahren zum Herstellen eines erfindungsgemässen Akkumulators. Das Verfahren umfasst die folgenden Schritte:
a) abwechslungsweises Einlegen einer positiven Elektrode mit einem Kontaktierungsanschluss und einer negativen Elektrode mit einem Kontaktierungsanschluss in ein Gehäuse, so dass sich der Kontaktierungsanschluss der positiven Elektrode in einer Ummantelung befindet und/oder so dass sich der Kontaktierungsanschluss der negativen Elektrode in einer Ummantelung befindet,
b) Einlegen eines Kontaktierungselementes auf den Kontaktierungsanschluss einer positiven Elektrode und / oder Einlegen eines Kontaktierungselementes auf den Kontaktierungsanschluss einer negativen Elektrode,
c) Wiederholen von Schritt b),
d) Herstellen mindestens einer Kontaktierungsvorrichtung durch Herstellen einer elektrisch leitenden Verbindung zwischen dem Kontaktierungselement und zwei benachbarten Kontaktierungsanschlüssen.

Ein solches Herstellungsverfahrens ist sehr gut und weitestgehend automatisierbar. Dies führt zu einer hohen und reproduzierbaren Qualität der Akkumulatoren bei attraktiven Herstellungskosten. Ausserdem ist ein solches Herstellungsverfahren auch für Kleinakkumulatoren gut geeignet.

Wenn sich sowohl die Kontaktierungsanschiüsse der positiven Elektroden in einer Ummantelung befinden, als auch die Kontaktierungsanschiüsse der negativen Elektroden in einer Ummantelung befinden, dann muss es ich nicht um ein und dieselbe Ummantelung handeln, sondern es kann sich um zwei räumlich getrennte Ummantelungen handeln.

In einer bevorzugten Ausführungsform umfasst der Schritt d) des Verfahrens zum Herstellen eines erfindungsgemässen Akkumulators ein Vernieten, Verschrauben und/oder Verschweissen eines Gegenlagers mit einem Stift.

Auf diese Weise kann die Kontaktierungsvorrichtung besonders einfach sowie mit guter Qualität und guten elektrischen Eigenschaften, insbesondere niedrigen Übergangswiderständen gefertigt werden.

In einer weiteren bevorzugten Ausführungsform umfasst das Verfahren zum Herstellen eines erfindungsgemässen Akkumulators zusätzlich einen oder mehrere der folgenden Schritte:
e) Ausschneiden und Abladieren der positiven Elektroden und der negativen Elektroden entsprechend vorgegebener Formen, und/oder
f) Zumindest partielles Beschichteten eines Stiftes mit einer Dichtung, und/oder
g) Verpressen eines Stiftes durch eine Gehäusedurchführung im Becherboden mit einem äusseren Kontakt, und/oder
h) Einschweissen der negativen Elektroden in jeweils einen Separator, und/oder
i) Trocknen, Zugeben von Elektrolyt, gasdichtes Montieren des Gehäusedeckels sowie Formieren des Akkumulators.

Das Ausschneiden und Abladieren der Elektroden gestattet eine optimale Passform der Elektroden in den Innenraum des Gehäuses und damit eine optimierte Kapazität des Akkumulators.

Das zumindest partielle Beschichten eines Stiftes mit einer Dichtung ermöglicht eine gasdichte Durchführung des Stiftes durch eine Gehäusedurchführung im Becherboden. Die Dichtung kann auch ein Klebstoff oder ein O-Ring sein.

Das Verpressen eines Stiftes durch eine Gehäusedurchführung im Becherboden mit einem äusseren Kontakt gestattet eine einfache Herstellung der Verbindung eines äusseren Kontaktes des Akkumulators mit der Kontaktierungsvorrichtung. Zudem kann so ein sehr platzsparender äusserer Kontakt hergestellt werden, der zudem direkt auf eine Leiterplatte montierbar ist.

Das Einschweissen der negativen Elektroden in jeweils einen Separator gestattet einen zuverlässigen und dauerhaften Betrieb des Akkumulators.

Das Trocknen, Zugeben von Elektrolyt, gasdichtes Montieren des Gehäusedeckels sowie Formieren des Akkumulators gestatten ebenfalls einen zuverlässigen und dauerhaften Betrieb des Akkumulators.

Mit der vorliegenden Erfindung gelingt es auch bei kleinen Bauformen, Akkumulatoren mit hoher Energiedichte und hoher Sicherheit gegen interne Kurzschlüsse und Dendritenbildung bereitzustellen, wobei die Akkumulatoren vor allem in medizintechnischen Geräten besonders vorteilhaft einsetzbar sind.

Aus der nachfolgenden Detailbeschreibung und der Gesamtheit der Patentansprüche ergeben sich weitere vorteilhafte Ausführungsformen und Merkmalskombinationen der Erfindung.

### Kurze Beschreibung der Zeichnungen

Die zur Erläuterung des Ausführungsbeispiels verwendeten Zeichnungen zeigen:
- Fig. 1: einen Querschnitt durch ein erstes Ausführungsbeispiel mit aufgeschraubter Hülse,
- Fig. 2: einen Querschnitt durch ein zweites Ausführungsbeispiel mit genieteter Hülse,
- Fig. 3: einen Querschnitt durch ein drittes Ausführungsbeispiel mit langen Hülsen und Verschraubung,
- Fig. 4: einen Querschnitt durch ein viertes Ausführungsbeispiel mit langen Hülsen und Vernietung,
- Fig. 5: einen Querschnitt durch ein fünftes Ausführungsbeispiel mit Graphitelektroden,
- Fig. 6: einen Gehäusebecher seitlich von unten,
- Fig. 7: einen Gehäusebecher von oben,
- Fig. 8: einen Gehäusebecher mit Öse seitlich von unten,
- Fig. 9: einen kundenspezifischen Gehäusebecher seitlich von oben.

Grundsätzlich sind in den Figuren gleiche Teile mit gleichen Bezugszeichen versehen.

### Wege zur Ausführung der Erfindung

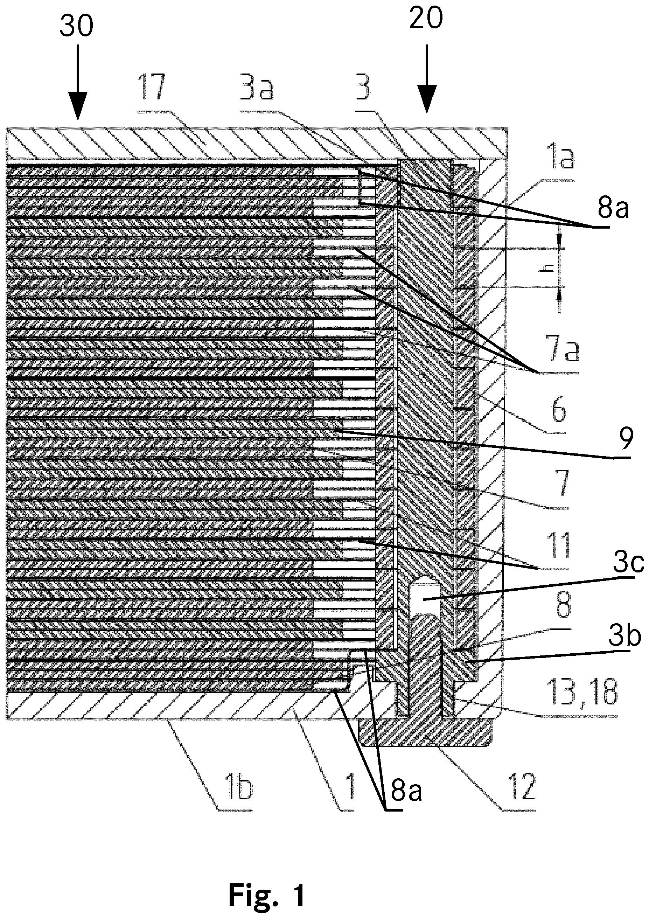

Fig. 1 zeigt einen Querschnitt durch ein erstes Ausführungsbeispiel des erfindungsgemässen Akkumulators im Bereich einer Kontaktierungsvorrichtung 20 für positive Elektroden. In einem Gehäusebecher 1 bestehend aus einer Becherwand 1a und einem Becherboden 1b befindet sich unter dem Gehäusedeckel 17 ein Stapel 30 umfassend beidseitig mit aktivem Material beschichtete positive Elektroden 7 und beidseitig mit aktivem Material beschichtete negative Elektroden 9. Die positiven geraden Kontaktierungsanschlüsse 7a der beidseitig beschichteten positiven Elektroden 7 führen zur Kontaktierungsvorrichtung 20. Des Weiteren umfasst der Stapel 30 eine Vielzahl von Separatoren 11. Jeweils ein Separator 11 ist zwischen einer positiven Elektrode 7 und einer negativen Elektrode 9 angeordnet. Zuunterst im Stapel 30 und direkt auf dem Becherboden 1b ist eine einseitig mit aktivem Material beschichtete positive Elektrode 8 angeordnet. Deren positiver abgewinkelter Kontaktierungsanschluss 8a führt zur Kontaktierungsvorrichtung 20.

Die Kontaktierungsvorrichtung 20 umfasst einen Stift 3 und eine Vielzahl von Kontaktierungselementen 6. Der Stift 3 weist im Bereich eines ersten, unteren Endes einen Flansch 3b auf. Der Flansch 3b stützt sich auf dem Becherboden 1b ab. Das erste, untere Ende des Stiftes 3 ist in einer Gehäusedurchführung 13 angeordnet, die sich im Becherboden 1b befindet. Eine Dichtung 18 stellt eine gasdichte Verbindung zwischen dem Stift 3 und dem Becherboden 1b her. Im Bereich des ersten, unteren Endes des Stiftes 3 ist im Stift 3 zudem eine Bohrung 3c angeordnet, in die ein äusserer Kontakt 12 eingepresst ist, der einen Teil der Unterseite des Becherbodens 1b überragt. Somit wird der Becherboden 1b im Bereich der Gehäusedurchführung 13 zwischen dem äusseren Kontakt 12 und dem Flansch 3b eingespannt, was zur gasdichten Abdichtung der Gehäusedurchführung 13 beiträgt. Aus diesem Grunde erstreckt sich das erste, untere Ende des Stiftes 3 auch nicht vollständig in die Gehäusedurchführung 13, sondern es verbleibt ein geringes Spiel zwischen der Stirnseite des ersten, unteren Ende des Stiftes 3 und dem äusseren Kontakt 12. Entsprechend erstreckt sich der äussere Kontakt 12 auch nicht vollständig in die Bohrung 3c, sondern es verbleibt auch hier ein gewisses Spiel. Zusätzlich kann sich in dem Bereich, in dem das Gehäuse zwischen dem äusseren Kontakt 12 und dem Flansch 3b des Stiftes 3 eingespannt ist, auch Dichtmaterial (nicht gezeigt) befinden. Der äussere Kontakt 12 ist mit dem Gerät (nicht dargestellt), in dem sich der erfindungsgemässe Akkumulator befindet, elektrisch verbindbar. In Fig. 1 stellt der äussere Kontakt 12 den Pluspol des Akkumulators dar. Die Kontaktierungsvorrichtung 20 ist nun wie folgt aufgebaut: Auf dem Flansch 3b des Stiftes 3 liegt zunächst der positive abgewinkelte Kontaktierungsanschluss 8a. Darauf folgt ein positiver gerader Kontaktierungsanschluss 7a. Nun folgen abwechselnd ein Kontaktierungselement 6 in Form einer Hülse und ein positiver gerader Kontaktierungsanschluss 7a. Zuoberst im Stapel 30 und direkt unter dem Gehäusedeckel 17 ist eine einseitig mit aktivem Material beschichtete positive Elektrode 8 angeordnet. Deren positiver abgewinkelter Kontaktierungsanschluss 8a führt ebenfalls zur Kontaktierungsvorrichtung 20 und liegt auf einem positiven geraden Kontaktierungsanschluss 7a auf. Darüber befindet sich ein letztes Kontaktierungselement 6, das im vorliegenden ersten Ausführungsbeispiel mit einem Gewinde 3a versehen ist und auf den ein mit einem entsprechenden Gewinde 3a versehener Stift 3 aufgeschraubt ist. Somit sind alle positiven Kontaktierungsanschiüsse 7a, 8a und die Kontaktierungselemente 6 zwischen dem Flansch 3b und dem als Gegenlager dienenden obersten aufgeschraubten Kontaktierungselement 6 derart eingespannt, dass sich ein möglichst kleiner Kontaktwiderstand zwischen den positiven Kontaktierungsanschlüssen 7a, 8a, den Kontaktierungselementen 6 und dem Stift 3 ergibt. Da der äussere Kontakt 12 mit dem Stift 3 verpresst ist, ist auch dieser Kontaktwiderstand minimal. Die Höhe h eines Kontaktierungselementes 6 ist so bemessen, dass sie dem Abstand zweier direkt benachbarter positiver gerader Kontaktierungsanschiüsse 7a entspricht. Beim vorliegenden ersten Ausführungsbeispiel entspricht die Höhe h des Kontaktierungselementes 6 der Dicke einer beidseitig beschichteten positiven Elektrode 7, zuzüglich der Dicke einer beidseitig beschichteten negativen Elektrode 9 und zuzüglich der Dicke von zwei Separatoren 11, sowie abzüglich der Dicke eines positiven geraden Kontaktierungsanschlusses 7a.

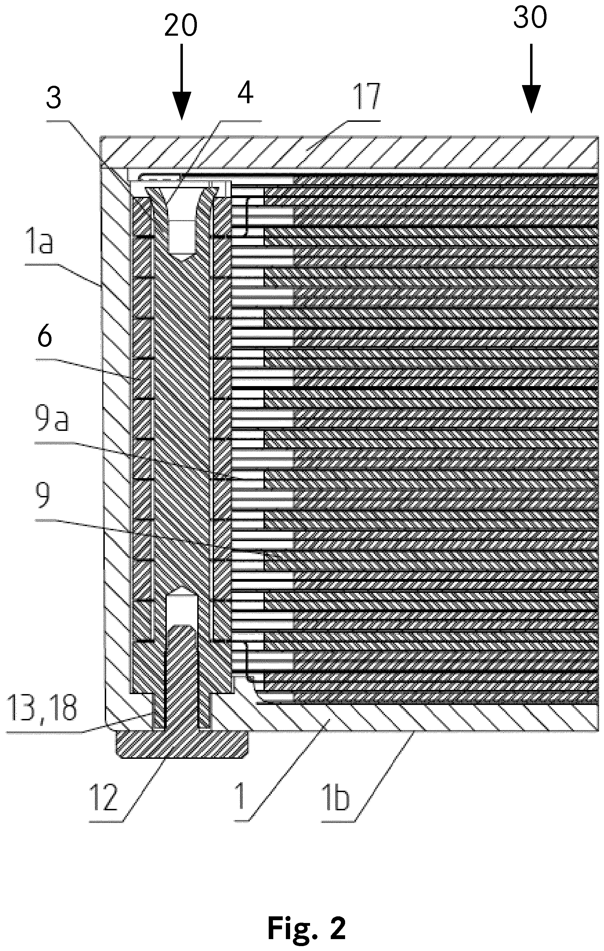

Fig. 2 zeigt einen Querschnitt durch ein zweites Ausführungsbeispiel des erfindungsgemässen Akkumulators im Bereich einer Kontaktierungsvorrichtung 20 für negative Elektroden. Es wird darauf verzichtet, die Merkmale des zweiten Ausführungsbeispiels zu wiederholen, die mit dem ersten Ausführungsbeispiel identisch sind. Im Unterschied zum ersten Ausführungsbeispiel werden beim zweiten Ausführungsbeispiel die negativen geraden Kontaktierungsanschiüsse 9a einer Vielzahl von beidseitig beschichteten negativen Elektroden 9 zur Kontaktierungsvorrichtung 20 geführt. In Fig. 2 stellt der äussere Kontakt 12 den Minuspol des Akkumulators dar. Die Kontaktierungsvorrichtung 20 ist wie folgt aufgebaut: Auf dem Flansch 3b des Stiftes 3 liegt zunächst der negative abgewinkelte Kontaktierungsanschluss einer einseitig beschichteten negativen Elektrode. Darauf folgt ein negativer gerader Kontaktierungsanschluss 9a. Nun folgen abwechselnd ein Kontaktierungselement 6 in Form einer Hülse und ein negativer gerader Kontaktierungsanschluss 9a. Zuoberst im Stapel 30 und direkt unter dem Gehäusedeckel 17 ist eine einseitig mit aktivem Material beschichtete negative Elektrode angeordnet. Deren negativer abgewinkelter Kontaktierungsanschluss führt zur Kontaktierungsvorrichtung 20 und liegt auf einem negativen geraden Kontaktierungsanschluss 9a auf. Darüber befindet sich ein letztes Kontaktierungselement 6. Im vorliegenden zweiten Ausführungsbeispiel ist eine Aussparung am zweiten, oberen Ende des Stiftes 3 vorgesehen zum Herstellen einer Nietverbindung 4. Bei der Herstellung eines Akkumulators gemäss dem zweiten Ausführungsbeispiel wird die Kontaktierungsvorrichtung 20 zusammengepresst und dann die Nietverbindung 4 hergestellt. Somit sind alle negativen Kontaktierungsanschiüsse und die Kontaktierungselemente 6 zwischen dem Flansch 3b und der als Gegenlager dienenden Nietverbindung 4 derart eingespannt, dass sich ein möglichst kleiner Kontaktwiderstand zwischen den negativen Kontaktierungsanschlüssen, den Kontaktierungselementen und dem Stift 3 ergibt.

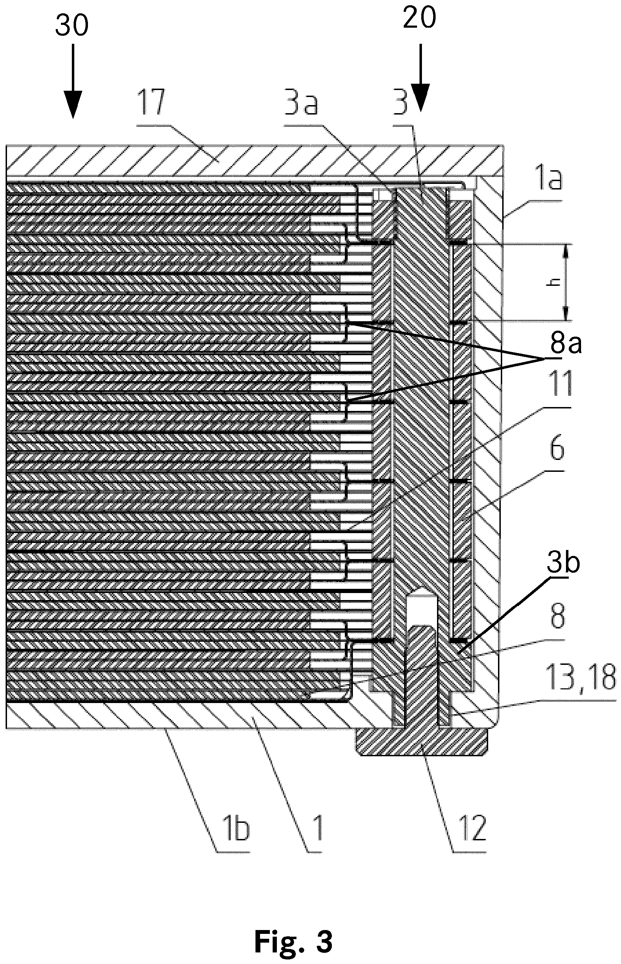

Fig. 3 zeigt einen Querschnitt durch ein drittes Ausführungsbeispiel des erfindungsgemässen Akkumulators im Bereich einer Kontaktierungsvorrichtung 20 für positive Elektroden. Das dritte Ausführungsbeispiel unterscheidet sich vom ersten Ausführungsbeispiel dadurch, dass die Höhe h eines Kontaktierungselementes 6 nun doppelt so gross ist und dass sich jeweils zwei positive abgewinkelte Kontaktierungsanschiüsse 8a zwischen zwei Kontaktierungselementen 6 befinden. Zwischen dem Flansch 3b und dem untersten Kontaktierungselement 6 befinden sich drei positive Kontaktierungsanschlüsse, und zwar neben den zwei positiven abgewinkelten Kontaktierungsanschlüssen 8a der beidseitig beschichteten positiven Elektroden auch noch ein positiver abgewinkelter Kontaktierungsanschluss der untersten einseitig beschichteten positiven Elektrode 8. Entsprechend sind auch über dem obersten Kontaktierungselement 6, das die Höhe h aufweist, insgesamt drei positive Kontaktierungsanschlüsse eingespannt. Beim vorliegenden dritten Ausführungsbeispiel entspricht die Höhe h der Kontaktierungselemente 6 der Dicke von zwei beidseitig beschichteten positiven Elektroden, zuzüglich der Dicke von zwei beidseitig beschichteten negativen Elektroden, zuzüglich der Dicke von vier Separatoren 11, sowie abzüglich der Dicke von zwei positiven abgewinkelten Kontaktierungsanschlüssen 8a. Die Kontaktierungselemente befinden sich wiederum zwischen zwei benachbarten positiven Kontaktierungsanschlüssen, wobei vorab jeweils zwei direkt benachbarte positive Kontaktierungsanschiüsse abgewinkelt und zusammengefasst wurden und somit einen neuen, gemeinsamen positiven abgewinkelten Kontaktierungsanschluss bilden.

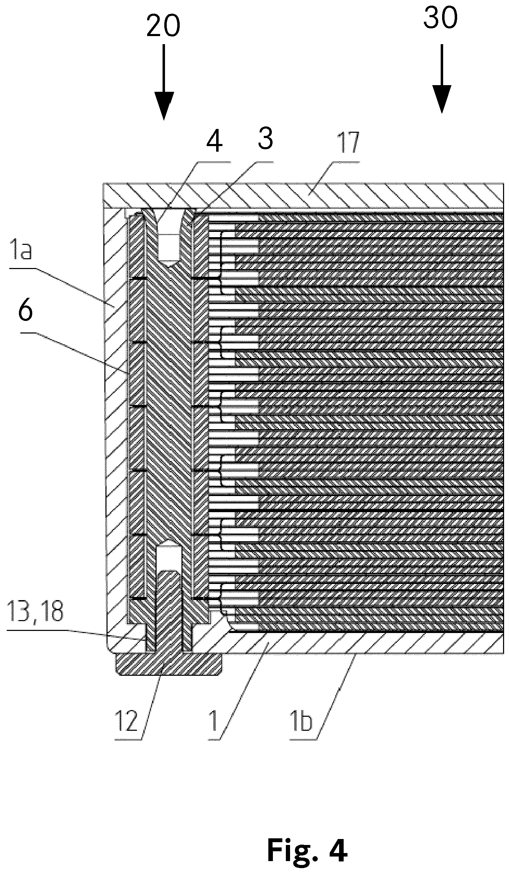

Fig. 4 zeigt einen Querschnitt durch ein viertes Ausführungsbeispiel des erfindungsgemässen Akkumulators im Bereich einer Kontaktierungsvorrichtung 20 für negative Elektroden. Analog zum vorhergehenden dritten Ausführungsbeispiel sind jetzt jeweils zwei direkt benachbarte negative abgewinkelte Kontaktierungsanschlüsse zusammengefasst, so dass die Kontaktierungselemente die doppelte Höhe haben verglichen mit den ersten beiden Ausführungsbeispielen. Im Unterschied zum dritten Ausführungsbeispiel werden bei dem vierten Ausführungsbeispiel die negativen Elektroden durch die Kontaktierungsvorrichtung 20 miteinander verbunden und eine Nietverbindung 4 kommt zu Einsatz, wie bei dem zweiten Ausführungsbeispiel.

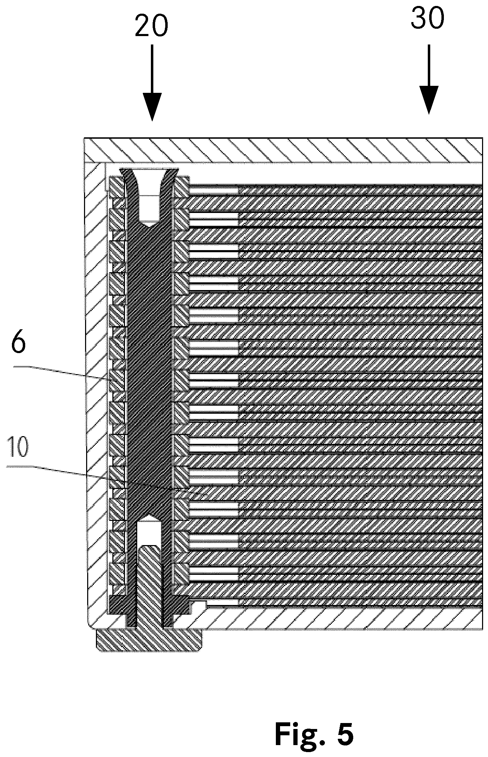

Fig. 5 zeigt einen Querschnitt durch ein fünftes Ausführungsbeispiel des erfindungsgemässen Akkumulators im Bereich einer Kontaktierungsvorrichtung 20 für negative Elektroden 10 mit Graphit. Die beidseitig beschichteten negativen Elektroden 10 sind frei von Metall und verfügen über ein Trägerband aus Graphit, dessen unbeschichteter Teil als negativer gerader Kontaktierungsanschluss dient und direkt zur Kontaktierungsvorrichtung 20 geführt wird. Folglich sind keine metallischen Ableitfahnen als Kontaktierungsanschluss zwischen dem aktiven Material der Elektrode und der Kontaktierungsvorrichtung 20 nötig. Allerdings können die Trägerbänder aus Graphit dicker sein als metallische Ableitfahnen, weswegen die Kontaktierungselemente 6 in diesem Ausführungsbeispiel eine entsprechend reduzierte Höhe haben können. In diesem Ausführungsbeispiel wird in der Kontaktierungsvorrichtung 20 eine Vernietung genutzt, aber eine Schraubverbindung anstelle der Nietverbindung, oder auch eine Schweissverbindung sind ebenso möglich.

Dem ersten bis fünften Ausführungsbeispielen ist gemeinsam, dass der Stift 3 einen möglichst kleinen Durchmesser im Bereich der Kontaktierungselemente hat.

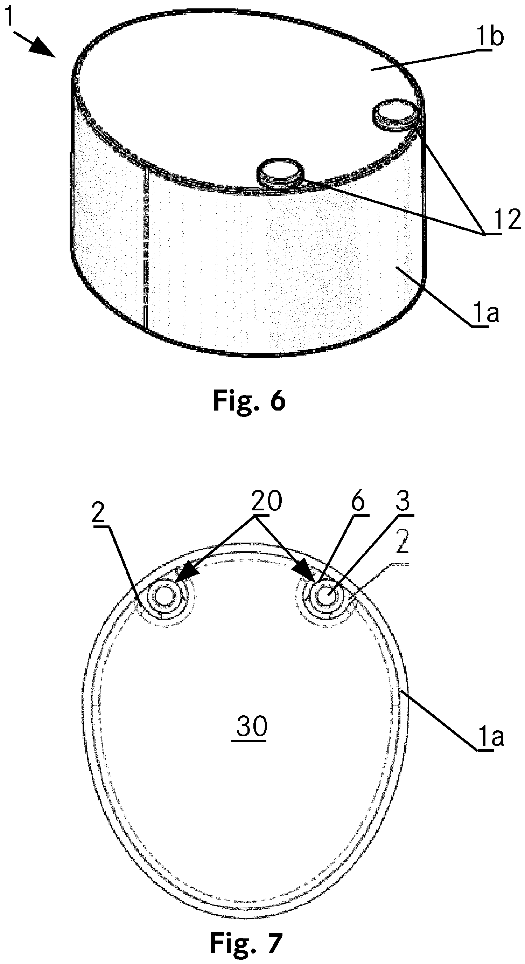

Fig. 6 zeigt einen Gehäusebecher 1 seitlich von unten. Aus dem Becherboden 1b ragen die beiden äusseren Kontakte 12 hervor. Sie stellen den Pluspol und den Minuspol des erfindungsgemässen Akkumulators dar und sind mit dem Gerät (nicht dargestellt), das der Akkumulator mit elektrischer Energie versorgen soll, kontaktierbar. Die äusseren Kontakte 12 befinden sich nahe der Becherwand 1 a. Der Gehäusebecher 1 hat eine ovale Grundform und ist ansonsten zylindrisch.

Fig. 7 zeigt den Gehäusebecher 1 von oben. Der Stapel 30 von positiven Elektroden und negativen Elektroden sowie Separatoren befindet sich im Gehäusebecher und nimmt fast das gesamte Volumen des Gehäusebechers in Anspruch. Lediglich die zwei in unmittelbarer Nähe der Becherwand 1a angeordneten Kontaktierungsvorrichtungen 20 sowie deren Ummantelungen 2 ragen in den Innenraum des Gehäusebechers hinein und reduzieren das für das aktive Material verfügbare Volumen. Jede der beiden Ummantelungen 2 wird jeweils aus der Becherwand 1a und zwei mit der Becherwand 1a verbundenen und aus ihr hervortretenden Vorsprüngen gebildet, so dass jede Kontaktierungsvorrichtung in Umfangsrichtung gemessen zu etwa 75% von der Ummantelung 2 umgeben ist. Die Ummantelung 2 liegt an der Kontaktierungsvorrichtung 2 an. Bei der Montage des erfindungsgemässen Akkumulators dient die Ummantelung 2 als Führung für die Kontaktierungsanschlüsse der Elektroden, die sich durch eine Öffnung in der Ummantelung 2 in die Kontaktierungsvorrichtung 20 erstrecken, und als Führung für die Kontaktierungselemente 6. Der Stift 3 kann zusätzlich bei der Montage als Führung für die Kontaktierungsanschlüsse und die Kontaktierungselemente dienen. Der Gehäusebecher 1 ist aus Kunststoff und kann zusammen mit den beiden Ummantelungen 2 einteilig hergestellt werden, beispielsweise per Spritzguss.

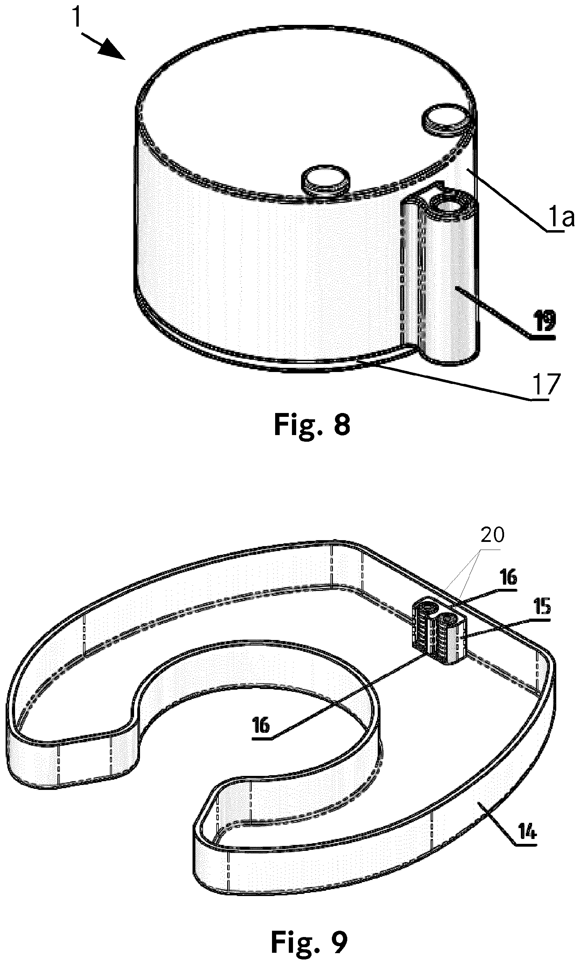

Fig. 8 zeigt einen Gehäusebecher 1 mit einer Öse 19 seitlich von unten. Die Öse 19 ist in diesem Ausführungsbeispiel an der Becherwand 1a angeordnet und kann der Befestigung in einem Gerät (nicht dargestellt) dienen. Der Gehäusebecher 1 ist aus Kunststoff und kann zusammen mit der Öse 19 einteilig hergestellt werden, beispielsweise per Spritzguss. Der Vollständigkeit halber ist noch zu erwähnen, dass der Gehäusebecher 1 mit einem Gehäusedeckel 17 verschlossen ist. Fig. 8 zeigt folglich einen fertigen erfindungsgemässen Akkumulator von aussen. Dabei kann sich im Inneren des Gehäusebechers 1 eine Anordnung gemäss des ersten, zweiten, dritten, vierten und/oder fünften Ausführungsbeispiels befinden, oder Kombinationen davon.

Fig. 9 zeigt einen kundenspezifischen U-förmigen Gehäusebecher 14 aus Metall seitlich von oben. Damit die Kontaktierungsanschlüsse gegenüber dem Gehäusebecher 14 elektrisch isoliert sind, befindet sich an einem Rand des Gehäusebechers 14 ein brillenförmiges Kunststoffteil 15 zur Aufnahme von zwei Kontaktierungsvorrichtungen 20. Das brillenförmige Kunststoffteil 15 kann durch eine äussere Isolation 16 besonders gegen den Gehäusebecher 14 isoliert sein. Der besseren Übersicht halber ist der Stapel 30 aus Elektroden und Separatoren in Fig. 9 nicht dargestellt.

Die im ersten bis fünften Ausführungsbeispiel erläuterten Varianten des erfindungsgemässen Akkumulators können in vielfältiger Weise miteinander kombiniert werden. Beispielsweise können das erste Ausführungsbeispiel und das zweite Ausführungsbeispiel miteinander zu einem weiteren Ausführungsbeispiel kombiniert werden, sodass innerhalb eines erfindungsgemässen Akkumulators sowohl die positiven Elektroden mit einer Kontaktierungsvorrichtung verbunden sind als auch die negativen Elektroden mit einer weiteren Kontaktierungsvorrichtung verbunden sind. Weitere Varianten entstehen dadurch, dass beide Kontaktierungsvorrichtungen an ihrem oberen Ende verschraubt (siehe Fig. 1), vernietet (siehe Fig. 2), oder verschweisst sein können. Analog lassen sich das dritte Ausführungsbeispiel und das vierte Ausführungsbeispiel kombinieren. Besonders vorteilhaft ist auch eine Kombination des fünften Ausführungsbeispiels (Graphit Elektroden) mit dem ersten Ausführungsbespiel. Wenn das fünfte Ausführungsbeispiel (Graphit-Elektroden) mit der Idee des vierten Ausführungsbeispiels (doppelte Höhe der Kontaktierungselemente) kombiniert wird, dann lässt sich das daraus entstehende Resultat auch gut mit dem dritten Ausführungsbeispiel kombinieren. Auf diese Art und Weise entsteht eine Vielzahl von Kombinationsmöglichkeiten, die hier nicht alle vollständig und explizit aufgezählt werden können, sich dem Fachmann aber ohne weiteres erschliessen.

Zusammenfassend ist festzustellen, dass die vorliegende Erfindung insbesondere für Kleinakkumulatoren eine erhebliche Verbesserung der Energiedichte und/oder Kapazität bietet, weil durch die platzsparende Ausführung der Kontaktierungsvorrichtung sowie deren Ummantelung mehr Platz für aktives Material zur Verfügung steht als bei herkömmlichen Akkumulatoren.

## Patentansprüche

1. Akkumulator, insbesondere Kleinakkumulator, zum Aufnehmen und Abgeben von elektrischer Energie, umfassend:
a) mindestens zwei positive Elektroden (7, 8) mit jeweils einem Kontaktierungsanschluss (7a, 8a),
b) mindestens zwei negative Elektroden (9, 10) mit jeweils einem Kontaktierungsanschluss (9a),
c) mindestens eine Kontaktierungsvorrichtung (20) mit mindestens einem Kontaktierungselement (6), das zwischen zwei benachbarten Kontaktierungsanschlüssen (7a, 8a, 9a) angeordnet ist und die zwei benachbarten Kontaktierungsanschiüsse (7a, 8a, 9a) elektrisch leitend miteinander verbindet,
d) ein Gehäuse, das die mindestens zwei positiven Elektroden (7, 8), die mindestens zwei negativen Elektroden (9, 10) und die mindestens eine Kontaktierungsvorrichtung(20) vollständig aufnimmt,
**dadurch gekennzeichnet, dass**
e) der Akkumulator mindestens eine Ummantelung (2) aufweist, die die mindestens eine Kontaktierungsvorrichtung (20) umgibt.

2. Akkumulator nach Anspruch 1, wobei die Kontaktierungsvorrichtung (20) eine Einspannvorrichtung aufweist, die das mindestens eine Kontaktierungselement (6) und die mindestens zwei benachbarten Kontaktierungsanschiüsse (7a, 8a, 9a) einspannt.

3. Akkumulator nach Anspruch 2, wobei
a) die mindestens zwei benachbarten Kontaktierungsanschlüsse (7a, 8a, 9a) jeweils ein Loch aufweisen,
b) das mindestens eine Kontaktierungselement (6) eine Hülse ist, und
c) die Einspannvorrichtung einen Stift (3) und ein Gegenlager (4, 6) aufweist, wobei der Stift (3) im Bereich eines ersten Endes einen Flansch (3b) und im Bereich eines zweiten Endes eine Verbindung (3a, 4) mit dem Gegenlager (4, 6) hat, und wobei der Stift (3) durch die Löcher der mindestens zwei benachbarten Kontaktierungsanschlüsse (7a, 8a, 9a) und durch das mindestens eine Kontaktierungselement (6) führt, und wobei sich die mindestens zwei benachbarten Kontaktierungsanschlüsse (7a, 8a, 9a) und das mindestens eine Kontaktierungselement (6) zwischen dem Flansch (3b) und dem Gegenlager (4, 6) befinden.

4. Akkumulator nach Anspruch 3, wobei die Verbindung des Stiftes (3) mit dem Gegenlager (4, 6) ein Gewinde (3a), eine Nietverbindung (4) und/oder eine Schweissverbindung ist.

5. Akkumulator nach einem der vorhergehenden Ansprüche, wobei die Kontaktierungsanschlüsse (7a, 8a) der mindestens zwei positiven Elektroden (7, 8) benachbart sind und/oder die Kontaktierungsanschlüsse (9a) der mindestens zwei negativen Elektroden (9) benachbart sind.

6. Akkumulator nach einem der vorhergehenden Ansprüche, wobei der Akkumulator zwei Kontaktierungsvorrichtungen (20) aufweist, die beide vollständig im Gehäuse angeordnet sind.

7. Akkumulator nach einem der vorhergehenden Ansprüche, wobei die Ummantelung (2) aus Kunststoff besteht.

8. Akkumulator nach einem der vorhergehenden Ansprüche, wobei das Gehäuse aus Kunststoff besteht.

9. Akkumulator nach einem der vorhergehenden Ansprüche, wobei das Gehäuse eine an einer Aussenseite des Gehäuses angeordnete Öse (19) aufweist.

10. Akkumulator nach einem der vorhergehenden Ansprüche, wobei mindestens eine der negativen Elektroden (10) einen Stromableiter aus einem elektrisch leitenden nicht-metallischen strukturellen Material aufweist.

11. Verwendung eines Akkumulators nach einem der vorhergehenden Ansprüche in einem medizintechnischen Gerät, insbesondere in einem Hörgerät, und im ganz Besonderen in einem in einen menschlichen Körper implantierbaren medizinischen Gerät.

12. Verfahren zum Herstellen eines Akkumulators nach einem der Ansprüche 1 bis 10, umfassend die Schritte:
a) abwechslungsweises Einlegen einer positiven Elektrode (7, 8) mit einem Kontaktierungsanschluss (7a, 8a) und einer negativen Elektrode (9, 10) mit einem Kontaktierungsanschluss (9a) in ein Gehäuse, so dass sich der Kontaktierungsanschluss (7a, 8a) der positiven Elektrode (7, 8) in einer Ummantelung (2) befindet und/oder so dass sich der Kontaktierungsanschluss (9) der negativen Elektrode (9, 10) in einer Ummantelung (2) befindet,
b) Einlegen eines Kontaktierungselementes (6) auf den Kontaktierungsanschluss (7a, 8a) einer positiven Elektrode (7, 8) und / oder Einlegen eines Kontaktierungselementes (6) auf den Kontaktierungsanschluss (9a) einer negativen Elektrode,
c) Wiederholen von Schritt b),
d) Herstellen mindestens einer Kontaktierungsvorrichtung 20 durch Herstellen einer elektrisch leitenden Verbindung zwischen dem Kontaktierungselement (6) und zwei benachbarten Kontaktierungsanschlüssen (7a, 8a, 9a).

13. Verfahren nach Anspruch 12, wobei der Schritt d) ein Vernieten, Verschrauben und/oder Verschweissen eines Gegenlagers (4, 6) mit einem Stift (3) umfasst.

14. Verfahren nach Anspruch 12 oder 13, zusätzlich umfassend einen oder mehrere der folgenden Schritte:
e) Ausschneiden und Abladieren der positiven Elektroden (7, 8) und der negativen Elektroden (9, 10) entsprechend vorgegebener Formen, und/oder
f) Zumindest partielles Beschichteten eines Stiftes (3) mit einer Dichtung (18), und/oder
g) Verpressen eines Stiftes (3) durch eine Gehäusedurchführung (13) im Becherboden (1b) mit einem äusseren Kontakt (12), und/oder
h) Einschweissen der negativen Elektroden (9, 10) in jeweils einen Separator (11), und/oder
i) Trocknen, Zugeben von Elektrolyt, gasdichtes Montieren des Gehäusedeckels (17) sowie Formieren des Akkumulators.
